(19) **Europäisches Patentamt**

**European Patent Office**

**Office européen des brevets**

(11) **EP 0 761 191 B1**

(12) **EUROPEAN PATENT SPECIFICATION**

(45) Date of publication and mention
of the grant of the patent:
**04.04.2001 Bulletin 2001/14**

(51) Int Cl.⁷: $A61F\ 13/15$, $A61F\ 5/48$

(21) Application number: **96117365.5**

(22) Date of filing: **10.09.1992**

(54) **Absorbent composites and absorbent articles containing same**

Zusammengesetzte absorbierende Materialien und diese enthaltende absorbierende Gegenstände

Composites absorbants et articles absorbants les contenant

(84) Designated Contracting States:
**BE DE ES FR GB IT NL SE**

(30) Priority: **11.09.1991 US 757787**
**26.06.1992 US 906001**
**11.09.1991 US 757760**

(43) Date of publication of application:
**12.03.1997 Bulletin 1997/11**

(62) Document number(s) of the earlier application(s) in
accordance with Art. 76 EPC:
**92115510.7 / 0 532 002**

(73) Proprietor: **KIMBERLY-CLARK WORLDWIDE,
INC.**
**Neenah, Wisconsin 54956 (US)**

(72) Inventors:
• **Byerly, Shannon Kathleen**
**Appleton, WI 54914 (US)**
• **Iwanski, David Gerard**
**Menasha, MI 54952 (US)**
• **Kellenberger, Stanley Roy**
**Appleton, WI 54911 (US)**
• **Qin, Jian**
**Appleton, WI 54914 (US)**
• **Shih-Schröder, Wen-Huey**
**Appleton, WI 54915 (US)**
• **Szymonski, Krzysztof Andrzej**
**Neenah, WI 54956 (US)**
• **Tsai, Chuan-Ling**
**Appleton, WI 54915 (US)**
• **Yarbrough, Sandra Marie**
**Appleton, WI 54914 (US)**

(74) Representative: **Davies, Christopher Robert**
**Frank B. Dehn & Co.**
**Patent and Trade Mark Attorneys,**
**179 Queen Victoria Street**
**London EC4V 4EL (GB)**

(56) References cited:
**EP-A- 0 198 683**      **EP-A- 0 339 461**
**EP-A- 0 422 504**      **EP-A- 0 443 627**
**US-A- 4 923 454**

**Description**

The invention refers to absorbent composites and absorbent articles containing same.

In the manufacture of disposable diapers, there is continual effort to improve the performance characteristics of the diaper. Although the structure of a diaper has many components, in many instances the in-use performance of the diaper is directly linked to the characteristics of the absorbent material contained within the diaper. Accordingly, diaper manufacturers strive to find ways of improving in-use absorbency in order to reduce the tendency of the diaper to leak.

One means of achieving this objective has been the extensive use of superabsorbent materials. Recent trends in commercial diaper design have been toward using more superabsorbent and less fiber in order to make the diaper thinner. Similarly in the literature, for example, U.S. Patent No. 5,021,050 to Iskra discloses a compressed composite structure of fibers and at least about 400 weight percent superabsorbent material, based on the weight of the fibers. However, notwithstanding the increase in total absorbent capacity contributed by the addition of larger amounts of superabsorbent material, such diapers often suffer from excessive leaking during use. Hence total absorbent capacity is only one factor to consider when selecting a superabsorbent material and designing a diaper or other absorbent article which will perform with fewer leaks during use.

Absorbent structures containing superabsorbent materials are disclosed in EP-A-0 339 461 and EP-A-0 443 627.

Therefore there is a need for a superabsorbent material that when used in a highly loaded absorbent composite, does not cause an unacceptable amount of leaking.

This object is solved by the absorbent composite of independent claim 1 and the absorbent article comprising an absorbent composite according to independent claim 32. Further advantageous features, aspects and details of this composite and this absorbent article are evident from the dependent claims, the description, examples and drawings. The invention also provides different applications of this article according to one of dependent claims 33 to 36.

It has been discovered that for diapers having a high loading of superabsorbent material (about 30 weight percent or greater as hereinafter defined), the superabsorbent material desirably has certain properties not previously appreciated and not necessary for superabsorbent materials used in conventional diapers, which contain less than about 20 weight percent superabsorbent material based on the combined weight of the fluff and the superabsorbent within the absorbent composite. The superabsorbent material properties to be considered are total absorbent capacity (hereinafter described as "Absorbent Capacity" or "AC"), resistance to deformation under load after the superabsorbent material has been partially saturated (hereinafter described as "Deformation Under Load" or "DUL"), the ability of the superabsorbent material to absorb an aqueous 0.9 weight percent NaCl solution while under a load of 0,395 Newtons per square centimeter (0.57 pounds per square inch), (hereinafter described as "Absorbency Under Load" or "AUL"), and the ability of the superabsorbent material to wick fluids away from the insult area.

For the purposes of this application, the ability of a superabsorbent material to wick fluids away from the insult area can be quantified in two manners. First, one can look at the distance a particular superabsorbent material can wick (transport) a fluid up an inclined trough (hereinafter described as "Wicking Index" or "WI"). Superabsorbent materials suitable for use in the present invention should have some minimum ability to transport (wick) a fluid. While the Wicking Index is good at setting a minimum acceptable performance characteristic of superabsorbent materials for use in the present invention, it may not be precise enough, when considered alone, to adequately predict relative performance of superabsorbent materials meeting this minimum acceptable performance characteristic. Nonetheless, when considered with other characteristics of a superabsorbent material, such as Deformation Under Load, or Absorbency Under Load, it is useful in predicting performance of superabsorbent material in the present invention.

A second manner of quantifying the ability of a superabsorbent material to wick fluids away from the insult area is to consider not only the distance a fluid is wicked but also the quantity of fluid wicked. This measurement of distance and quantity is made not only when the superabsorbent is relatively dry, as when the first insult occurs, but after the superabsorbent material has been partially saturated. Making the distance/quantity measurements after the superabsorbent material is partially swollen is believed to approximate the behavior of the superabsorbent material for insults occurring after an initial insult. As will be described in greater detail below, the Wicking Parameter (hereinafter sometimes referred to as "WP") is a test capable of measuring and quantifying the wicking distance/quantity capability of a superabsorbent material at various levels of saturation. Wicking Parameter alone has been found to be predictive of the relative performance of superabsorbent material in composites of the present invention.

The methods for determining Absorbent Capacity, Deformation Under Load, Absorbency Under Load, Wicking Index, and Wicking Parameter will be described in detail hereinafter.

While not being bound to any theory, it is believed that the Deformation Under Load, Absorbency Under Load, Wicking Index and Wicking Parameter characteristics of a superabsorbent material are important for the performance of a highly loaded superabsorbent composite because of the greater number of particle-to-particle interactions created by the higher concentration of superabsorbent particles within the absorbent composite. Highly deformed superabsorbent particles will tend to block wicking channels which initially exist between the particles. Hence, resistance to deformation becomes much more important in such highly loaded superabsorbent composites than in conventional absorbent composites. Similarly, particles which cannot absorb a liquid under a load may not, in use, be able to expand from a dry state sufficiently to maintain wicking channels which initially exist between the particles and/or create new wicking channels. Hence, a high Absorbency Under Load becomes more important in such highly loaded superabsorbent composites than in conventional absorbent composites. Thus, Deformation Under Load evaluates the ability of a superabsorbent material to maintain wicking channels after the superabsorbent material is swollen, and Absorbency Under Load evaluates the ability of a superabsorbent material to maintain and/or create wicking channels as the superabsorbent material swells.

When considering the properties mentioned above, it is possible to have a relatively low total Absorbent Capacity and still have adequate performance in use if certain of the other properties discussed above are sufficiently high. This will be explained in greater detail in connection with the examples. It will be appreciated that many other factors, not a part of this invention, also greatly impact product performance, such as product design, fit, the conditions under which the product is used, etc.

Hence in one aspect, the invention resides in an absorbent composite comprising a matrix of fibers and superabsorbent material having at least about 30 weight percent superabsorbent material based on the combined weight of the fibers of the matrix and the superabsorbent material, said superabsorbent material having a Deformation Under Load of about 0.60 millimeter or less and a Wicking Index of about 10 centimeters or greater. An Absorbent Capacity of about 28 grams per gram or greater is preferred.

In another aspect, the invention resides in an absorbent article comprising a liquid-permeable facing material, a liquid impermeable backing material, and an absorbent composite sandwiched between the facing material and the backing material, said absorbent composite comprising a matrix of fibers and superabsorbent material having at least about 30 weight percent superabsorbent material based on the combined weight of the fibers of the matrix and the superabsorbent material, said superabsorbent material having a Deformation Under Load of about 0.60 millimeters or less and a Wicking Index of about 10 centimeters or greater. An Absorbent Capacity of about 28 grams per gram or greater is preferred. The absorbent article can also contain a number of other components well known in the art such as transfer layers, leg elastics, waist elastics, tapes, and the like.

In another aspect, the invention resides in an absorbent composite comprising a matrix of fibers and superabsorbent material having at least about 30 weight percent superabsorbent material based on the combined weight of the fibers of the matrix and the superabsorbent material, said superabsorbent material having a Wicking Parameter of about 700 or greater.

In another aspect, the invention resides in an absorbent article comprising a liquid-permeable facing material, a liquid impermeable backing material, and an absorbent composite sandwiched between the facing material and the backing material, said absorbent composite comprising a matrix of fibers and superabsorbent material having at least about 30 weight percent superabsorbent material based on the combined weight of the fibers of the matrix and the superabsorbent material, said superabsorbent material having a Wicking Parameter of at least about 700 or greater. The absorbent article can also contain a number of other components well known in the art, such as transfer layers, leg elastics, waist elastics, tapes, and the like.

In another aspect, the invention resides in an absorbent composite comprising a matrix of fibers and superabsorbent material having at least about 30 weight percent superabsorbent material based on the combined weight of the fibers of the matrix and the superabsorbent material, said superabsorbent material having an Absorbency Under Load of about 13 or greater.

In another aspect, the invention resides in an absorbent article comprising a liquid-permeable facing material, a liquid impermeable backing material, and an absorbent composite sandwiched between the facing material and the backing material, said absorbent composite comprising a matrix of fibers and superabsorbent material having at least about 30 weight percent superabsorbent material based on the combined weight of the fibers of the matrix and the superabsorbent material, said superabsorbent material having an Absorbency Under Load of about 13 or greater. The absorbent article can also contain a number of other components well known in the art, such as transfer layers, leg elastics, waist elastics, tapes, and the like.

Although this invention is primarily described in connection with disposable diapers, it is also applicable to other products having an absorbent composite, particularly those which are rapidly exposed to large amounts of liquid, such as training pants, incontinence garments, bed pads, and the like.

In certain aspects of this invention, the Deformation Under Load is about 0.6 millimeter or less, preferably about 0.5 millimeter or less, and more preferably about 0.4 millimeter or less, and still more preferably about 0.3 millimeter or less. A suitable range is from about 0.3 to about 0.6 millimeter or less. The Wicking Index is about 10 centimeters or greater, preferably about 12 centimeters or greater, more preferably about 15 centimeters or greater, and most preferably about 18 centimeters or greater. A suitable range is from about 12 to about 19 centimeters or greater. The Absorbent Capacity is preferably about 28 grams per gram or greater, more preferably about 32 grams per gram or greater, still more preferably about 36 grams per gram or greater, and most preferably about 40 grams per gram or greater. A suitable range is from about 28 to about 41 grams per gram or greater.

The Wicking Parameter is about 700 or greater, preferably about 800 or greater, more preferably about 850 or greater, and most preferably about 900 or greater.

The Absorbency Under Load is about 13 or greater, preferably about 17 or greater, more preferably about 20 or greater, and most preferably about 25 or greater. A suitable range is from about 13 to about 25 or greater.

The amount of the superabsorbent material in the absorbent composite is about 30 weight percent or greater, preferably about 40 weight percent or greater, and more preferably about 50 or 60 weight percent or greater. One embodiment of the absorbent composite of this invention, as used in a diaper, contains about 50 weight percent superabsorbent material. Such a diaper is disclosed in commonly assigned copending patent application "Absorbent Article", E.P. Application No. 92115530, filed of even date in the names of W.D. Hanson et al.. However, the amount of superabsorbent material can be from about 30, 40, or 50 weight percent to about 60, 70, 80, or 90 weight percent, even 100 weight percent if the superabsorbent material is in the form of fibers or filaments. The distribution of the superabsorbent material within the absorbent composite can be uniform or nonuniform, such as by being layered or otherwise non uniformly placed within the absorbent composite.

For purposes herein, the term "superabsorbent material" is any material which is capable of absorbing or gelling at least 10 times its weight, preferably 15 times its weight, of body exudate or a suitable aqueous solution such as 0.9 weight percent solution of sodium chloride in distilled water. Such materials include, but are not limited to, hydrogel-forming polymers which are alkali metal salts of: poly(acrylic acid); poly(methacrylic acid); copolymers of acrylic and methacrylic acid with acrylamide, vinyl alcohol, acrylic esters, vinyl pyrrolidone, vinyl sulfonic acids, vinyl acetate, vinyl morpholinone and vinyl ethers; hydrolyzed acrylonitrile grafted starch; acrylic acid grafted starch; maleic anhydride copolymers with ethylene, isobutylene, styrene, and vinyl ethers; polysaccharides such as carboxymethyl starch, carboxymethyl cellulose, methyl cellulose, and hydroxypropyl cellulose; poly(acrylamides); poly(vinyl pyrrolidone); poly(vinyl morpholinone); poly(vinyl pyridine); and copolymers and mixtures of any of the above and the like. The hydrogel-forming polymers are preferably lightly crosslinked to render them substantially water-insoluble. Crosslinking may be achieved by irradiation or by covalent, ionic, van der Waals attractions, or hydrogen bonding interactions, for example. A preferable superabsorbent material is a lightly crosslinked hydrocolloid. The superabsorbent materials can be in any form suitable for use in absorbent structures or composites, including particles, fibers, bicomponent fibers, filaments, flakes, spheres, and the like.

The fibers useful for the absorbent composite of this invention are preferably in the form of an airlaid batt of comminuted wood pulp (fluff), the formation of which is well known in the art of diaper manufacture. Although comminuted wood pulp is preferred, other cellulosic fibers, such as cotton linters, can also be used. Suitable synthetic fibers include, without limitation, fibers of polyethylene, polypropylene, polyesters, copolymers of polyesters and polyamides, bicomponent fibers, and the like. Mixtures of natural and synthetic fibers can also be used. The fibers used to form the matrix of the absorbent composite are generally hydrophilic or rendered hydrophilic through a suitable surface treatment. The preferred wood pulp fluff is produced by fiberizing bleached northern or southern softwood kraft pulp, although hardwood pulps and blends of hardwood and softwood pulps can also be used. By way of illustration, a blend of hardwood and softwood pulps can have a weight ratio of softwood pulp to hardwood pulp of from about 1:3 to about 20:1.

The absorbent composite of this invention comprises a porous matrix of fibers and superabsorbent material dispersed among the interfiber spaces and/or fiber pores or between or on fiber sheets. Thus, as used herein, the term "matrix of fibers" refers to any fibrous structure which contains a superabsorbent material. This includes, without limitation, airlaid batts as described above as well as fibrous webs on which a superabsorbent material is contained, and fibrous sheets between which a superabsorbent material is contained. While particulate superabsorbent material is preferred because of its commercial availability, the superabsorbent material can also be in the form of continuous or discontinuous fibers. The formation of the absorbent composite can be accomplished in any number of ways, such as are currently used in the manufacture of commercially available diapers. A suitable example of one means of forming the absorbent composite is disclosed in U.S. Patent No. 4,927,582 to Bryson et al.

Because the superabsorbent material in the absorbent composite is present in relatively high proportions, the absorbent composite of the present invention can be relatively thin while still functioning in an acceptable manner. Advantageously, the absorbent composites of this invention can have an average thickness of less than about .51 cm (about 0.2 inch) and preferably less than about .38 cm (about 0.15 inch). As used herein, the average thickness is the average of a statistically significant number of thickness measurements taken under an applied load of 13.8 kPa (0.2 pounds per square inch). The number of thickness measurements taken depends on the size and uniformity of the absorbent composite, and must be sufficient to represent the average thickness of the entire absorbent composite.

Figure 1 is a perspective view of the apparatus for measuring the Deformation Under Load.

Figure 2 is a sectional view of the sample cup used for measuring the Deformation Under Load.

Figure 3 is a sectional view of the sample while being partially saturated in preparation for measuring the Deformation Under Load.

Figure 4 is a sectional view of the apparatus illustrated in Figures 1-3 while measuring the Deformation Under Load.

Figure 5 is a perspective view of the apparatus used to determine the Wicking Index.

Figure 6 is a side view of the apparatus of Figure 5.

Figure 7 is an exploded perspective view of the apparatus used to measure Wicking Parameter.

Figure 8 is a cross-sectional view of the apparatus used to measure Absorbency Under Load.

In order to determine the Deformation Under Load and the Wicking Index for the superabsorbent materials of this invention, as will be hereinafter described, a synthetic urine was used as the absorbed fluid to closely approximate in use performance in diapers. The synthetic urine composition referenced herein comprises 1.0 gram methyl paraben, 0.68 grams monobasic potassium phosphate ($KH_2PO_4$), 0.31 grams monobasic calcium phosphate monohydrate ($CaH_4(PO_4)2 \times H_2O$), 0.48 gram magnesium sulphate heptahydrate ($MgSO_4 \times 7H_2O$), 1.33 grams potassium sulphate ($K_2SO_4$), 1.24 grams tribasic sodium phosphate dodecahydrate ($Na_3PO_4 \times 12H_2O$), 4.44 grams sodium chloride (NaCl), 3.16 grams potassium chloride (KCl), 8.56 grams of urea ($CO(NH_2)_2$), 1.0 gram Germall 115 preservative (commercially available from Santell Chemical Company, Chicago, Illinois), and 0.1 gram Pluronic 10R8 surfactant (a nonionic surfactant commercially available from BASF-Wyandotte Corporation). The components are added to 900 milliliters of distilled water in the order given and each dissolved before the next component is added. The solution is finally diluted to 1 liter and has a surface tension in the range of 54-58 dynes per centimeter.

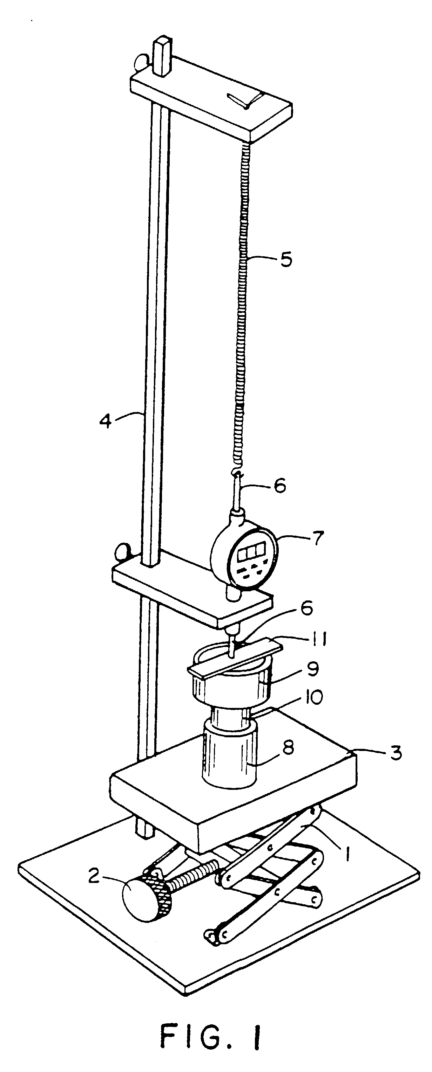

Referring now to the Drawing, the invention will be further described in more detail. As previously discussed, the Deformation Under Load is an important factor in the various aspects of this invention. The Deformation Under Load is essentially a measure of a gelled superabsorbent material's ability to resist compression deformation under a controlled load. Briefly, the test involves the incomplete saturation of a superabsorbent material with a fixed amount of synthetic urine, compressing the superabsorbent material under a light load, and then measuring the deformation of the sample under a heavier load, all under ambient conditions. Referring to figures 1-4, the test apparatus and procedure will be described in detail.

Figure 1 is a perspective view of the test apparatus during testing. Shown is a laboratory jack 1 having an adjustable knob 2 for raising and lowering the platform 3. A laboratory stand 4 supports a suspension spring 5 connected to the probe 6 of a modified thickness meter (described below). The housing 7 of the thickness meter is rigidly affixed to and supported by the laboratory stand. The probe extends through the housing of the thickness meter, which detects any movement of the probe. Also shown is a plastic sample cup 8, a plastic weight cup 9 having a cylindrical foot 10, and a glass slide 11.

The modified thickness meter, which is used to measure the deformation of the sample under load, is a Mitutoyo Digimatic Indicator, IDC Series 543, Model 543-180, having a range of 0 - 1.27 cm (0-0.5 inch) and an accuracy of 1.27 μm (0.00005 inch) (Mitutoyo Corporation, 31-19, Shiba 5-chome, Minato-ku, Tokyo 108, Japan). As supplied from Mitutoyo Corporation, the thickness meter contains a spring attached to the probe within the meter housing. This spring is removed to provide a free falling probe, which has a downward force of about 27 grams. In addition, the cap over the top of the probe located on the top of the meter housing is also removed to enable attachment of the probe to the suspension spring 5 (Available from McMaster-Carr Supply Co., Chicago, Illinois, Item No. 9640K41), which serves to counter or reduce the downward force of the probe to about 1 gram, ± 0.5 gram. A wire hook can be glued to the top of the probe for attachment to the suspension spring. The bottom tip of the probe is also provided with an extension needle (Mitutoyo Corporation, Part No. 131279) to enable the probe to be inserted into the sample cup.

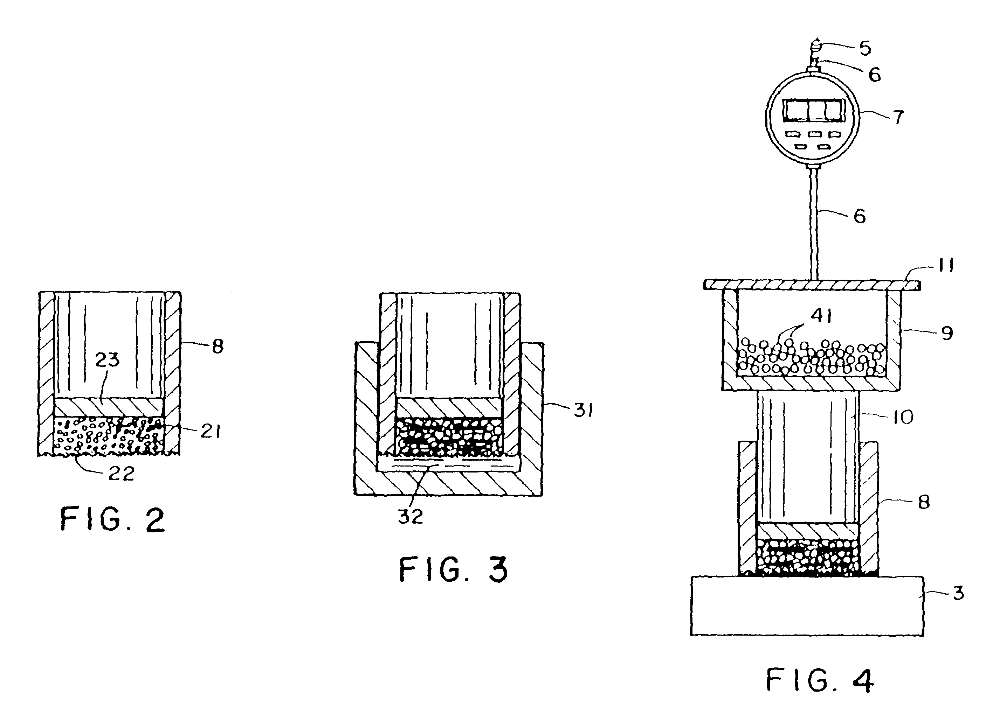

Figure 2 is a sectional view of the sample cup 8 into which the superabsorbent particles 21 to be tested are placed. The sample cup is a plastic cylinder having a 2.54 cm (1 inch) inside diameter and an outside diameter of 3.18 cm (1.25 inch). The bottom of the cup is formed by adhering (gluing) a 100 mesh metal screen 22, having 150 μm openings, to the end of the cylinder. A 0.1600 gram (±.0005 gram) sample of the superabsorbent material, which has been sieved to a particle size between 300 and 600 μm, is placed into the sample cup and evenly spread over the screen bottom. (Fibrous superabsorbent materials need not be sieved.) The sample is then covered with a plastic spacer disc 23 (having a diameter of 2.51 - 2.53 cm (0.990-0.995 inch)) to protect the sample from being disturbed during the test.

The sample cup is then slowly lowered into a plastic reservoir cup 31 containing 4.00 grams of synthetic urine 32 as illustrated in the sectional view of Figure 3, being careful not to disrupt the superabsorbent material with escaping air. The inside diameter of the reservoir cup is only slightly greater than 3.18 cm (1.25 inch) in order to provide a snug fit between the sample cup and the reservoir cup sufficient to prevent the synthetic urine from escaping between the sample cup and the reservoir cup. The sample cup is lowered to the bottom of the reservoir cup such that the synthetic urine is gently forced up through the screen to evenly contact the superabsorbent material. The sample cup remains inside the reservoir cup for 30 minutes to ensure that all of the synthetic urine is absorbed by the sample.

The sample cup is removed from the reservoir cup and placed on the platform 3 of the laboratory jack as illustrated in the sectional view of Figure 4. The plastic weight cup 9 having a cylindrical foot 10 is used to apply a known load to the sample. The cylindrical foot has an outside diameter of 2.51 - 2.53 cm (0.990-0.995 inch). The bottom of the foot is solid. The weight cup is also provided with a glass slide 11 which bridges the open top of the weight cup and provides a flat surface against which the probe 6 of the thickness meter is positioned. The combined total weight of the weight cup, including the foot and the glass slide, and the spacer disc in the sample cup, is 100 grams. If the total weight falls short of 100 grams, some lead shot 41 can be placed inside the weight cup to bring the combined weight up to the 100 gram level.

When testing the sample, the foot of the weight cup is placed inside the sample cup and the platform is raised up until the probe of the thickness meter contacts the glass slide and then is raised up slightly further to give the probe enough play to return toward its initial position during the subsequent test. For most materials, the probe should be raised about 3 millimeters above its normal resting point. The load on the sample at this point is 2.07 kPa (0.3 pounds per square inch). The thickness meter is then set to zero, and 200 grams of lead shot 41 or other suitable weight are added to the weight cup, bringing the load up to 6.21 kPa (300 grams or 0.9 pounds per square inch). The downward distance of travel of the probe from the zero point, which is read after the rate of change is less than 0.006 millimeters in two minutes, expressed in millimeters, is the Deformation Under Load of the sample. Normally the reading can be taken within 10 to 20 minutes.

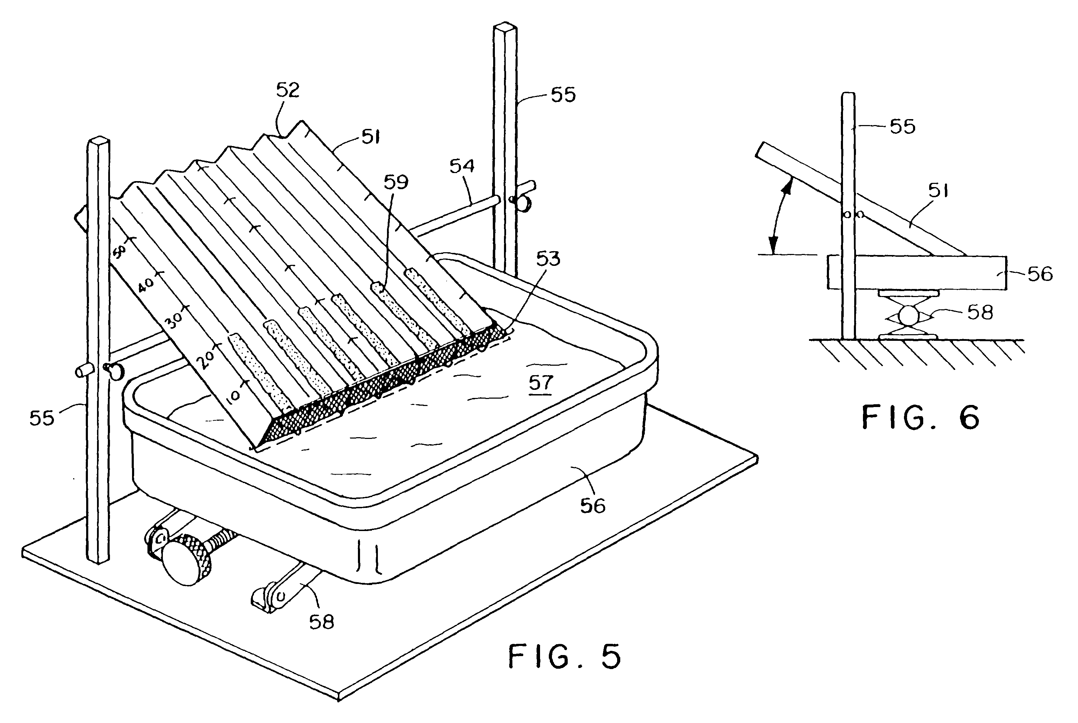

The Wicking Index is a measure of a superabsorbent material's ability to wick away fluid without the aid of a fibrous network. This property can be especially important for absorbent composites containing high loadings of superabsorbent material and relatively low amounts of fluff. Briefly, the test is performed at ambient conditions by spreading out an amount of superabsorbent material into a continuous bed of particles within an inclined trough and contacting the bottom of the continuous bed of superabsorbent particles with synthetic urine and measuring the distance the synthetic urine has been wicked after 60 minutes. Referring to Figures 5 and 6, the apparatus and method for determining the Wicking Index will be further described.

Figure 5 is a perspective view of the apparatus for carrying out the Wicking Index measurement. Shown is a trough sheet 51 made of rigid metal (18 gauge 304 stainless steel having an extra low carbon surface and a grade 2B finish) and containing six trough channels 52. Each trough channel has 90° side angles and must be at least 20 centimeters in length. The peak-to-peak width of each trough channel is 5.5 centimeters, The depth of each trough is 2.75 centimeters. The trough sheet is enclosed on one end with a 100 mesh stainless steel screen 53 (having 150 μm openings) which has been soldered to the trough sheet and serves to contain the superabsorbent material being tested while permitting the synthetic urine to pass through. The trough length is incremented in 0.5 centimeter units beginning with 0 centimeters at the enclosed screen end. A cross bar 54 attached to the trough sheet provide means for supporting the trough sheet using laboratory stands 55 with suitable clamps or other attachment means. A fluid reservoir pan 56, having 7.6 cm (3 inch) high sidewalls and a length of about 30.5 cm (about 12 inches) and a width of about 45.7 cm (about 18 inches), is sufficiently large to enclose the screen end of the trough sheet and contains a sufficient amount of synthetic urine 57 to carry out the test as described below. Two laboratory jacks 58 provide a means for raising or lowering the reservoir pan under the trough sheet for fluid level adjustment. Also shown are six individual particle beds 59 of superabsorbent material evenly spread out a length of 20 centimeters within the trough channels.

Figure 6 is a side view of the apparatus of Figure 5 in position during testing. Shown is the trough sheet 51 supported by the laboratory stands at an angle of 20° from horizontal as indicated by the double arrow. The laboratory jacks 58 support the reservoir pan 56 in position to enable the superabsorbent samples within the individual trough channels to wick the fluid from the reservoir pan.

To carry out the Wicking Index measurement, the trough sheet is supported above the fluid reservoir pan at an angle of 20° from horizontal. The screened end of the trough sheet, which is the lowermost end, is level side to side. Before starting the test, the bottom (screened end) of the trough sheet should be approximately 5.1 - 7.6 cm (2-3 inches) above the bottom of the reservoir pan, which should be level. Individual samples of superabsorbent material (1.00 gram each, ± 0.005 gram, sieved to 300-600 μm particle size) are evenly sprinkled in separate trough channels between the 0 and 20 centimeter graduations, assuring an even distribution. (For samples which have a Wicking Index greater than 20 centimeters, the bed of particles can be spread over a distance greater than 20 centimeters using a proportionally greater sample size.) Using the squared off end of a .8 cm (5/16 inch) wide spatula, each superabsorbent particle bed is smoothed out and more evenly spread within its trough channel. Synthetic urine, colored with FD&C Blue Dye #1 to enhance measurement readings without altering the surface tension beyond the target range of $54\text{-}58 \cdot 10^{-5}$ N per centimeter, is poured into the reservoir pan making sure that the trough channels do not get wet. A fluid level in the reservoir pan of about 2 centimeters has been found to be adequate for testing six samples simultaneously. The reservoir pan is carefully raised to a level where a visual approximation of simultaneous contact of the fluid with all of the trough channels will occur. Adjustment of the trough sheet to the fluid can be done at this time by either raising or lowering one of the side arm clamps on the laboratory support stands while maintaining the 20° angle. The reservoir pan is further raised until the fluid level is about 0.5 centimeter above the trough bottom to assure a continual availability of fluid to the superabsorbent particle bed. As soon as the fluid wets the stainless steel screen, timing of the test is begun. After 60 minutes, the distance the fluid has been wicked is observed. This is the Wicking Index, expressed as centimeters rounded to the nearest one-half centimeter.

As used herein, the Absorbent Capacity is a measure of the absorbent capacity of the superabsorbent material retained after being subjected to centrifugation under controlled conditions. It is measured by placing 0.200 grams of the sample material to be tested (moisture content of less than 5 weight percent) into a water-permeable bag which will contain the sample while allowing the test solution (0.9 percent NaCl solution) to be freely absorbed by the sample. A heat-sealable tea bag material (grade 542, commercially available from Kimberly-Clark Corporation, Neenah, Wisconsin) works well for most applications. The bag is formed by folding a 12.7 cm (5 inches) by 7.6 cm (3 inches) sample of the bag material in half and heat sealing two of the open edges to form a 6.35 x 7.6 cm (2.5 x 3 inch) rectangular pouch. The heat seals should be about .635 cm (about 0.25 inch) inside the edge of the material. After the sample is placed in the pouch, the remaining open edge of the pouch is also heat-sealed. Empty bags are also made to be tested with the sample bags as controls. Three sample bags are tested for each superabsorbent material.

The sealed bags are placed between two Teflon® coated fiberglass screens having .635 cm (1/4 inch) openings (Taconic Plastics, Inc., Petersburg, N.Y.) and submerged in a pan of 0.9 percent NaCl solution at 23°C ± 1.11°C (73.4°F. ± 2°F.), making sure that the screens are held down until the bags are completely wetted. After wetting, the samples remain in the solution for 30 minutes, at which time they are removed from the solution and temporarily laid on a nonabsorbent flat surface. The wet bags are then placed into the basket of a suitable centrifuge capable of subjecting the samples to a g-force of 350. (A suitable centrifuge is a Clay Adams Dynac II, model #0103, having a water collection basket, digital rpm gauge, and machined drainage basket adapted to hold and drain the flat bag samples.) The samples must be placed in opposing positions within the centrifuge to balance the basket when spinning. The bags are centrifuged at a target of 1600 rpm, but within the range of 1500-1900 rpm, for 3 minutes (target g-force of 350). The bags are removed and weighed, with the empty bags (controls) being weighed first, followed by the bags containing superabsorbent material. The amount of fluid absorbed and retained by the superabsorbent material, taking into account the fluid retained by the bag material alone, is the Absorbent Capacity of the superabsorbent material, expressed as grams of fluid per gram of superabsorbent material.

The Wicking Parameter is a measure of the ability of a superabsorbent material to wick away (transport) a quantity of fluid without the aid of a fibrous network. The Wicking Parameter quantifies not only the distance that a superabsorbent material can wick a liquid but also the quantity of liquid wicked. This property can be especially important for absorbent composites containing high loadings of superabsorbent material and relatively low amounts of fluff. Briefly, the test is performed at ambient conditions by forming the superabsorbent material to be tested into a continuous bed of particles. The particles may then be preconditioned by allowing them to become partially swollen in an aqueous 0.9 weight percent sodium chloride solution. The continuous bed of particles is then raised to an incline with the bottom of the continuous bed of superabsorbent material in contact with an aqueous solution containing 0.9 weight percent sodium chloride. The distance and amount of fluid transported by the bed of particles is measured over a two-hour time period.

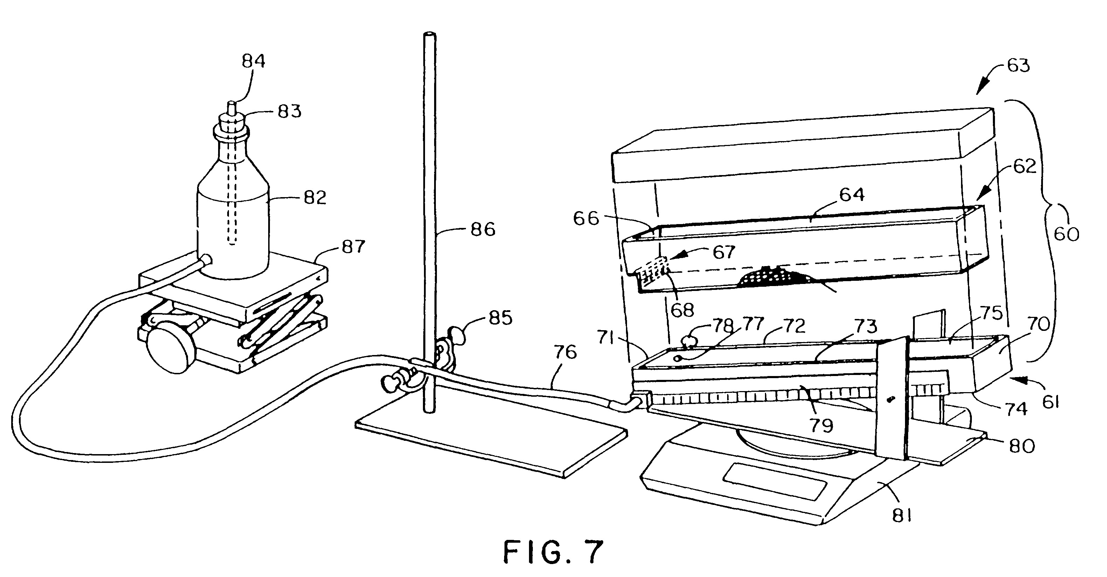

Referring to Fig. 7, the apparatus and method for determining the Wicking Parameter will be further described.

Fig. 7 is an exploded perspective view of the apparatus used for carrying out the Wicking Parameter measurement. Fig. 7 illustrates test container 60 comprising a holding chamber 61, a testing chamber 62, and a cover 63. Testing chamber 62 is a rectangular chamber 2.54 cm (1 inch) wide, 35.56 cm (14 inches) long, and 3.81 cm (1.5 inches) deep (internal dimensions). The testing chamber 62 is suitably formed from a clear material such as an acrylic resin commercially available under the trademark Lucite$^{TM}$ (0.635 cm (0.25 inch) thick). The top 64 of testing chamber 62 is open. The bottom 65 of testing chamber 62 is formed from a 100 mesh metal screen. The metal screen is adhered to the material forming the sides and ends of testing chamber 62. One longitudinal end 66 of the test chamber 62 is formed by a piece of Lucite$^{TM}$ material (or other suitable material) which is dimensioned such that the testing chamber 62 defines a 2.54 cm (1 inch) wide by 0.9525 cm (0.375 inch) deep opening 67. The opening 67 is covered with a 100 mesh screen 68. The mesh screen 68 is suitably adhered to the Lucite$^{TM}$ material forming test chamber 62 around the periphery of opening 67. Bottom 65 and screen 68 are adhered at their juncture or are formed as a single, integral piece.

Holding chamber 61 comprises longitudinal ends 70, 71, lateral sides 72, 73, and bottom 74. Holding chamber 61 is suitably formed from a clear acrylic resin such as Lucite$^{TM}$ material (0.635 cm (0.25 inch) thick). Longitudinal ends 70, 71, lateral sides 72, 73, and bottom 74 of holding chamber 61 define a top opening 75. When the testing chamber 62 is formed from 0.635 cm (0.25 inch) thick Lucite$^{TM}$ material, holding chamber 61 is dimensioned to form a chamber 3.81 cm (1.5 inches) wide, 36.83 cm (14.5 inches) long and 2.54 cm (1.0 inch) deep (internal dimensions). In any event, holding chamber 61 is internally dimensioned so that testing chamber 62 can just pass into, and snugly fit within, the interior of holding chamber 61.

Holding chamber 61 defines a 0.3175 cm (0.125 inch) diameter opening by which supply tube 76 (0.4763 cm (0.1875 inch) diameter opening) is in communication with the interior of holding chamber 61. Holding chamber 61 further comprises a threaded opening 77 containing a screw 78. Screw 78 is configured such that one end of it can pass through threaded opening 77 and contact the test chamber 62 when it is present in holding chamber 61. A clear plastic ruler 79 is suitably attached to side 73 of holding chamber 61 to make distance measurements (as described below) more easily.

Cover 63 is similarly formed from a clear acrylic resin such as Lucite[TM] and is dimensioned to cover top opening 75 of holding chamber 61 when testing chamber 62 is present therein. Cover 63 defines an interior chamber 3.81 cm (1.5 inches) wide, 36.83 cm (14.5 inches) long and 1.4288 cm (0.5625 inch) deep.

A quantity of the superabsorbent material to be tested is sieved to provide a sample having a particle size of 300 to 600 μm. Three grams of the sieved superabsorbent material is evenly distributed on the mesh screen forming bottom 65 of testing chamber 62. If the superabsorbent material is to be preconditioned by allowing it to become partially swollen in a 0.9 weight percent aqueous sodium chloride solution, the amount of an aqueous sodium chloride solution necessary to reach the desired degree of preconditioning is placed in the bottom of holding chamber 61 or, alternatively, in the chamber defined by cover 63. At this point, the opening through which supply tube 76 communicates with the interior of holding chamber 61 is blocked to prevent the sodium chloride solution from passing out of holding chamber 61 and holding chamber 61 is horizontal. The testing chamber 62 containing the superabsorbent material to be tested is then carefully lowered into holding chamber 61 and allowed to absorb the liquid therein for a period of time of 30 minutes. It is desirable to maintain the thickness of the preconditioned, partially swollen superabsorbent material as even as possible.

The test container 60 is then placed on incline base 80 which is configured such that the bottom 74 of holding chamber 61 forms an incline angle of 20 degrees above horizontal. Incline base 80 in turn rests on electronic balance (scale) 81. A reservoir for liquid is provided comprising an aspirator bottle 82 including a rubber stopper 83 and an aspirator tube 84. The aspirator bottle 82 is connected by supply tube 76 to holding chamber 61. Supply tube 76 is supported by clamp 85 which is attached to laboratory stand 86 in order to minimize the effect of movement of supply tube 76 on electronic balance 81 during testing. The aspirator bottle rests on laboratory jack 87. The aspirator bottle is filled with an aqueous solution containing 0.9 weight percent sodium chloride. The saline solution in aspirator bottle 82 is colored with FD & C blue dye no. 1 to enhance measurement readings.

To start the testing procedure, testing chamber 62 and cover 63 are removed from holding chamber 61 which remains in place on incline base 80. The aspirator bottle is raised on laboratory jack 87 until the saline solution contained in aspirator bottle 82 fills the lower end (about 0.64 cm (about 0.25 inch)) of holding chamber 61 to a 0.635 cm (0.25 inch) depth at its deepest point. At this point, the testing chamber 62 is placed in the holding chamber 61 but is held out of contact with the saline solution present in holding chamber 61 by screw 78. Specifically, screw 78 is passed through threaded opening 77 until it contacts the side of test chamber 62. The force exerted by screw 78 presses test chamber 62 against holding chamber 61 and prevents the test chamber 62 from completely entering holding chamber 61. Cover 63 is then placed on holding chamber 61. Balance 81 is then zeroed, and the bottom end of screen 68 is lowered into the saline solution by releasing the force exerted by screw 78. The junction of screen 68 and bottom 65, and the superabsorbent material located generally thereat, contact the saline solution. Nonetheless, the screw 78 is employed such that the bottom 65 does not touch the bottom of holding chamber 61. In this way, the saline solution cannot wick at the interface of testing chamber 62 and holding chamber 61. The saline solution is fed at a constant hydrostatic head from the aspirator bottle 82 into the lower end of holding chamber 61. The progress of the saline solution in centimeters and the increase in weight, as registered by balance 81, as a function of time, are recorded for a period of two hours.

As discussed above, the superabsorbent materials to be tested are, for some of the tests, preconditioned by allowing them to become partially swollen in an aqueous 0.9 weight percent sodium chloride solution. Preconditioning refers to the weight of saline solution (0.9 weight percent) made available to the superabsorbent material on a gram of saline per gram of superabsorbent material basis. For each superabsorbent material tested, the above test is repeated at the following preconditioning (partial swelling) levels (not including moisture inherently present in the superabsorbent material, typically less than about 10 weight percent): 0 grams per gram, 10 grams per gram, 15 grams per gram, 20 grams per gram, 25 grams per gram, and 30 grams per gram. The Wicking Parameter is calculated according to the following formula:

$$WP = \sum_{i=0}^{n-1} \frac{1}{2} \left( \sqrt{WD_i \times WC_i} + \sqrt{WD_{i+1} \times WC_{i+1}} \right) \times \left( S_{i+1} - S_i \right)$$

wherein WP is the Wicking Parameter, n is the number of different preconditioning levels (6 according to the above test method), WD is the Wicking Distance defined as the furthest distance, in centimeters, the blue saline solution from aspirator bottle 82 wicked along the superabsorbent material bed in test chamber 62 at the end of two hours; WC is the Wicking Capacity defined as the amount of fluid, in grams, drawn, transported or absorbed by the superabsorbent material, as registered by balance 81, at the end of two hours, and S is the sample preconditioning level in grams per gram.

Thus, assuming the following test results for a superabsorbent material:

| Preconditioning (g/g) | WD (cm) | WC (g) |
|---|---|---|
| 0 | 20 | 74.9 |
| 10 | 25.2 | 65.3 |
| 15 | 26.5 | 57.8 |
| 20 | 28.0 | 48.1 |
| 25 | 24.5 | 44.0 |
| 30 | 20.0 | 26.5 |

The calculated Wicking Parameter is 1,099.

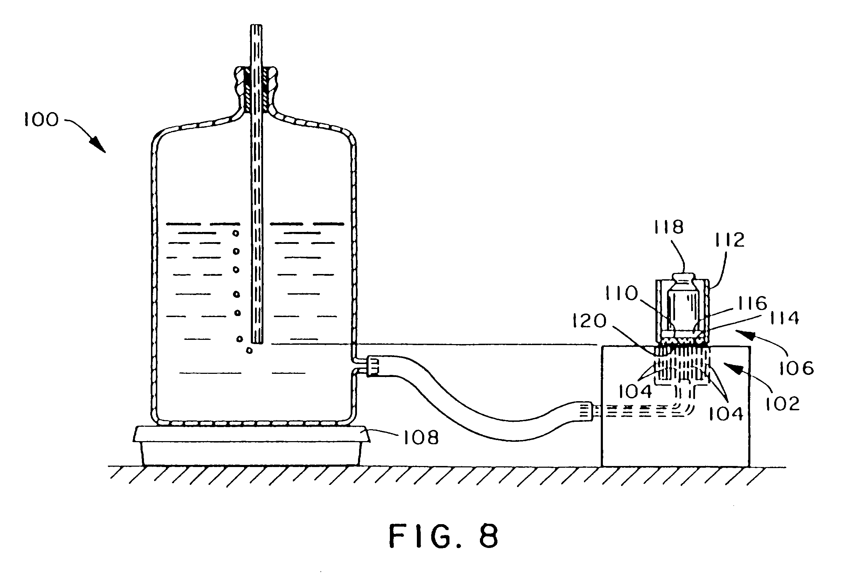

The Absorbency Under Load test is a measure of the ability of a superabsorbent material to absorb a liquid while the superabsorbent material is under a restraining load. The test can best be understood by reference to Fig. 8 which is a cross-sectional view of the equipment used to measure the AUL of a superabsorbent material. Referring to Fig. 8, a demand absorbency tester (DAT) 100 is used, which is similar to a GATS (gravimetric absorbency test system), available from M/K Systems, Danners, MA, as well as a system described by Lichstein in pages 129-142 of the INDA Technological Symposium Proceedings, March 1974. A porous plate 102 is used having ports 104 confined within the 2.5 centimeter area covered, in use, by the Absorbency Under Load apparatus 106. An electrobalance 108 is used to measure the flow of the test fluid (an aqueous solution containing 0.9 weight percent sodium chloride) into the superabsorbent material 110. The AUL apparatus 106 used to contain the superabsorbent material is made from 1 inch (2.54 centimeter), inside diameter, thermoplastic tubing 112 machined-out slightly to be sure of concentricity. One hundred mesh stainless steel wire cloth 114 is adhesively attached to the bottom of tubing 112. Alternatively, the steel wire cloth 114 can be heated in a flame until red hot, after which the tubing 112 is held onto the cloth until cooled. Care must be taken to maintain a flat, smooth bottom and not distort the inside of the tubing 112. A 4.4 gram piston 116 is made from 2.54 cm (1 inch) solid material (e.g., plexiglass) and is machined to closely fit, without binding, in the tubing 112. A 200 gram weight 118 (outer diameter 2.4892 cm (0.98 inch)) is used to provide 39,500 dynes per square centimeter (about 0.57 psi) restraining load on the superabsorbent material. A sample corresponding to a layer of at least about 300 grams per square meter (0.16 grams) of superabsorbent material is utilized for testing the Absorbency Under Load. The sample is taken from superabsorbent material which is prescreened through U.S. standard #30 mesh and retained on U.S. standard #50 mesh. The superabsorbent material, therefore, has a particle size of between 300 and 600 μm. The particles can be prescreened by hand or automatically with, for example, a Ro-Tap Mechanical Sieve Shaker Model B available from W. S. Tyler, Inc., Mentor, Ohio.

The test is initiated by placing a 3 centimeter diameter GF/A glass filter paper 120 onto the plate 102 (the paper is sized to be larger than the internal diameter and smaller than the outside diameter of the tubing 112) to ensure good contact while eliminating evaporation over the ports 104 of the demand absorbency tester 100 and then allowing saturation to occur. The desired amount of superabsorbent material 110 (0.16 grams) is weighed onto weigh paper and placed on the wire cloth 114 at the bottom of the tubing 112. The tubing 112 is shaken to level the superabsorbent material on the wire cloth 114. Care is taken to be sure no superabsorbent material is clinging to the wall of the tubing 112. After carefully placing the piston 116 and weight 118 on the superabsorbent material to be tested, the apparatus 106 is placed on the glass filter paper 120. The amount of fluid picked up is monitored as a function of time either directly by hand, with a strip chart recorder, or directly into a data acquisition or personal computer system.

The amount of fluid pick-up measured after 1 hour is the AUL value and is reported in grams of test liquid absorbed per gram of superabsorbent material as determined before starting the test procedure. A check can be made to ensure the accuracy of the test. The apparatus 106 can be weighed before and after the test with a difference in weight equaling the fluid pick-up.

Examples

In order to illustrate the advantages of this invention, diapers having absorbent composites containing 10 grams of fluff and 10 grams of superabsorbent material were tested in use to determine their effectiveness in reducing leaks. The structure of the test diapers was as disclosed the aforementioned commonly assigned copending E.P. patent application No. 92115530 filed of even date but did not include a surge material. More specifically, 60 babies (30 male and 30 female) were recruited. The care giver was given 10 diapers containing a particular superabsorbent sample and instructed to use the diapers for two days under normal conditions and to indicate if the diaper leaked or not. Diapers containing bowel movements were excluded from consideration when evaluating the data. A total of 600 diapers were used for each superabsorbent sample.

The performance evaluation for the various samples is based on the leakage of the test diaper relative to a control diaper in the same use test. Because use tests conducted at different times with different babies will often yield different absolute leakage numbers, relative results within a given use test, as compared to a control are a more representative indicator of the effectiveness of the superabsorbent being tested. The control diaper for all testing was a commercially available diaper having a superabsorbent material loading of about 12-15 weight percent (HUGGIES[TM] Supertrim, manufactured by Kimberly-Clark Corporation, Neenah, Wisconsin). A performance rating of "+" means that no significant difference (within 95% confidence limits) in overall leakage was observed relative to the control. A performance rating of "O" means that there was a statistically significant difference in the overall percent leakage relative to the control, but the difference was less than 6 percentage points. A performance rating of "-" means an unacceptable amount of overall leakage relative to the control (greater than 6 percentage points).

The results of diaper leakage testing are presented below in Table 1. As indicated, some superabsorbent samples were use-tested twice. The sample identifications, including the manufacturer, are as follows: Sample 1- Partial sodium salt of crosslinked poly-2-propenoic acid (Dow Chemical Company, Midland, Michigan, No.40453.00, lot 105); Sample 2- Starch grafted crosslinked sodium salt of poly(acrylic acid) (Hoechst Celanese Corporation, Portsmouth, Virginia, No. S-243); Sample 3- Starch grafted crosslinked sodium salt of poly(acrylic acid) (Hoechst Celanese, Sanyo IM5000S); Sample 4- Starch grafted sodium salt of poly(acrylic acid) (Hoechst Celanese S-241); Sample 5- Polyacrylate/polyalcohol (Stockhausen, Inc., Greensboro, North Carolina, No. W45926); Sample 6- Starch grafted crosslinked sodium salt of poly(acrylic acid) (Hoechst Celanese, No. IM3900); Sample 7-Partial sodium salt of crosslinked poly-2-propenoic acid (Dow 40453.00, lot 111-2); Sample 8-Polyacrylate/polyalcohol (Stockhausen, No. W45353); Sample 9-Polyacrylate/polyalcohol (Stockhausen, Favor SAB 835); Sample 10-Partial sodium salt of crosslinked polypropenoic acid (Dow, Drytech 534); Sample 11-Starch grafted crosslinked sodium salt of poly(acrylic acid) (Hoechst Celanese, S-242; Sample 12-Polyacrylate/polyalcohol (Stockhausen, No. W45939); and Sample 13-Starch grafted crosslinked sodium salt of poly(acrylic acid) (Hoechst Celanese IMIOOOP).

Table I1

| Sample | AC | DUL | WI | AUL | WP | Performance |
|---|---|---|---|---|---|---|
| 1 | 29 | 0.42 | 13.0 | 13.2 | 813 | + |
| 2 | 41 | 0.38 | 13.0 | 16.6 | 901 | + |
| 3 | 35 | 0.38 | 18.0 | 9.4 | 1081 | + |
| 4 | 37 | 0.43 | 15.0 | 21.9 | 921 | + |
| 5 | 33 | 0.54 | 16.5 | 19.9 | 746 | 0 |
| 6 | 34 | 0.34 | 18.5 | 9.8 | 1099 | + |
| 7 | 28 | 0.61 | 12.5 | 17.0 | 834 | 0 |
| 8 | 39 | 0.45 | 12.5 | 14.3 | 843 | 0 |
| 9[2] | 30 | 1.02 | 15.0 | 9.4 | 556 | 0 |
| | 27 | 1.08 | 14.0 | 8.7 | 572 | - |
| 10[3] | 31 | 0.79 | 13.0 | 10 | 675 | - |
| | 31 | 0.78 | 8.5 | 9.4 | 663 | - |
| 11[4] | 42 | 0.66 | 12.5 | 10.5 | 672 | - |
| 12 | 32 | 0.58 | 16.0 | 23.3 | 689 | 0 |
| 13[5] | 51 | 0.29 | 5.5 | 6.4 | 390 | - |

[1]Data reported is generally the average of multiple tests

[2]Use tested twice employing superabsorbents having the same commercial designation but two different lot numbers. As one use test gave a performance rating of 0, and the other a performance rating of -, the superabsorbent is considered unacceptable

[3]Use tested twice employing superabsorbents having the same commercial designation but two different lot numbers. Both samples tested gave a performance rating of -

[4] The superabsorbent material was removed from a test diaper before the DUL and WP were measured. This may be necessary if the diaper manufacturing process changes the properties of the superabsorbent material. WI and AUL will likely be similarly affected.

[5]Performance was tested under similar conditions but different than the conditions used for the other samples.

As can be seen from reference to Table 1, Deformation Under Load and Wicking Index are major factors when evaluating or predicting the performance of a superabsorbent material in a high superabsorbent concentration composite. Superabsorbent material exhibiting a Deformation Under Load of about 0.6 millimeters or less, and a Wicking Index of about 10 centimeters or greater, gives an acceptable performance rating of + or 0. Minimal Absorbent Capacity (about 28 grams per gram) can be acceptable when the Deformation Under Load is about 0.6 millimeters or less and the Wicking Index is about 10 centimeters or greater.

As can also be seen from reference to Table 1, Deformation Under Load and Wicking Index are not the only properties which are suitable for predicting the performance of a superabsorbent material in use. Superabsorbent material having a Wicking Parameter of about 700 or greater is also generally found to provide acceptable in-use performance (performance rating of + or 0). Moreover, it is seen that, of the superabsorbent materials tested, those exhibiting the best performance (a + rating) have a higher Wicking Parameter than those superabsorbents exhibiting acceptable performance but having a lower performance rating (rating of 0). Thus, the Wicking Parameter is able not only to predict which superabsorbent materials will perform at an acceptable level but to distinguish among those superabsorbent materials exhibiting an acceptable level of performance to determine which may provide the best performance.

Similarly, of the superabsorbent materials tested, those having an Absorbency Under Load value of greater than about 13 are generally found to be acceptable performers. Those having an Absorbency Under Load value of less than about 13 are generally not acceptable performers. While exceptions to this generalization exist, e.g., sample nos. 3 and 6, it is believed that those samples exhibit acceptable performance due to their extremely low DUL values, high Wicking Index, and high Wicking Parameter.

Thus, it is seen that the present invention provides alternative ways of selecting from among superabsorbent materials to choose those superabsorbent materials which are best suited for providing good performance in an absorbent composite containing a relatively high concentration of superabsorbent material.

It will be appreciated that the foregoing examples, provided for purposes of illustration, are not to be taken as limiting the scope of this invention.

To summarize the above, described is an absorbent composite comprising a matrix of fibers and superabsorbent material having at least about 30 weight percent superabsorbent material based on the combined weight of the fibers and the superabsorbent material, said superabsorbent material having a Deformation Under Load of about 0.60 millimeters or less preferably about 0.5 mm or less, more preferably about 0.4 mm or less, and particularly about 0.3 mm or less and a wicking Index of about 10 centimeters or greater.

The absorbent composite can preferably have a Deformation Under Load of from 0.3 to 0.6 millimeter.

The absorbent composite furthermore may comprise a matrix of fibers and superabsorbent material having at least about 50 weight percent superabsorbent material based on the combined weight of the fibers and the superabsorbent material, said superabsorbent material having a Deformation Under Load of about 0.6 millimeters or less and a Wicking Index of about 12 centimeters or greater.

It is preferred that the absorbent composite has a Wicking Index of about 15 centimeters or greater, more preferably of about 18 centimeters or greater.

According to another aspect, there is provided an absorbent composite comprising a matrix of fibers and superabsorbent material having from 30 to 60 weight percent superabsorbent material based on the combined weight of the fibers and the superabsorbent material, said superabsorbent material having an Absorbent Capacity of from 28 to 41 grams per gram, a Deformation Under Load of from 0.3 to 0.6 millimeter, and a Wicking Index of from 12 to 19 centimeters.

A still further aspect refers to an absorbent composite comprising a matrix of fibers and superabsorbent material having at least about 30 weight percent superabsorbent material based on the combined weight of the fibers and the superabsorbent material, said superabsorbent material having a Wicking Parameter of about 700 or greater, preferably of about 800 or greater, more preferably of about 850 or greater, and particularly about 900 or greater.

A further aspect relates to an absorbent composite comprising a matrix of fibers and superabsorbent material having at least about 30 weight percent superabsorbent material based on the combined weight of the fiber and the superabsorbent material, said superabsorbent material having an Absorbency Under Load of about 13 or greater, preferably of about 17 or greater, more preferably of about 20 or greater, and particularly of about 25 or greater.

The absorbent composite according to the present invention has preferably about 40 weight percent or more superabsorbent material, more preferably about 50 weight percent or more superabsorbent material, even more preferably about 60 weight percent or more superabsorbent material, still even more preferably about 70 weight percent or more superabsorbent material, particularly about 80 weight percent or more superabsorbent material, and more particularly about 90 weight percent or more superabsorbent material.

The present invention furthermore relates to an absorbent article comprising a liquid-permeable facing material, a liquid impermeable backing material, and an absorbent composite according to the invention sandwiched between the facing material and the backing material.

The absorbent article may be a disposable diaper, a training pant, an incontinence garment or a bed pad.

## EP 0 761 191 B1

**Claims**

1. An absorbent composite comprising a matrix of fibers and superabsorbent material, having at least about 30 weight percent superabsorbent material based on the combined weight of the fiber and the superabsorbent material, characterised in that the superabsorbent material has a Wicking Parameter of about 700 or greater.

2. An absorbent composite according to claim 1, wherein the superabsorbent material has a Wicking Parameter of about 800 or greater.

3. An absorbent composite according to claim 1 or 2, wherein the superabsorbent material has a Wicking Parameter of about 850 or greater.

4. An absorbent composite according to any preceding claim, wherein the superabsorbent material has a Wicking Parameter of about 900 or greater.

5. An absorbent composite according to any preceding claim, wherein the superabsorbent material has an Absorbency Under Load of about 13 or greater.

6. An absorbent composite according to any preceding claim, wherein the superabsorbent material has an Absorbency Under Load of about 17 or greater.

7. An absorbent composite according to claim 6, wherein the superabsorbent material has a Wicking Index of about 10 centimeters or greater.

8. An absorbent composite according to claim 6 or 7, wherein the superabsorbent material has a Deformation Under Load of about 0.6 millimeters or less.

9. An absorbent composite according to any preceding claim, wherein the superabsorbent material has a Deformation Under Load of about 0.6 millimeters or less and a Wicking Index of about 10 centimeters or greater.

10. An absorbent composite according to any preceding claim comprising at least about 50 weight percent superabsorbent material based on the combined weight of the fibers and the superabsorbent material, the superabsorbent material having a Deformation Under Load of about 0.6 millimeters or less and a Wicking Index of about 12 centimeters or greater.

11. An absorbent composite according to any of claims 1 to 9 comprising from 30 to 60 weight percent superabsorbent material based on the combined weight of the fibers and the superabsorbent material, the superabsorbent material having an Absorbent Capacity of from 28 to 41 grams per gram, a Deformation Under Load of from 0.3 to 0.6 millimeters, and a Wicking Index of from 12 to 19 centimeters.

12. An absorbent composite according to any preceding claim, wherein the superabsorbent material has an Absorbency Under Load of about 20 or greater.

13. An absorbent composite according to any preceding claim, wherein the superabsorbent material has an Absorbency Under Load of about 25 or greater.

14. An absorbent composite according to any preceding claim, wherein the superabsorbent material has a Deformation Under Load of about 0.5 millimeters or less.

15. An absorbent composite according to any preceding claim, wherein the superabsorbent material has a Deformation Under Load of about 0.4 millimeters or less.

16. The absorbent composite according to any preceding claim except claim 11, wherein the superabsorbent material has a Deformation Under Load of about 0.3 millimeters or less.

17. An absorbent composite according to any of claims 1 to 15, wherein the superabsorbent material has a Deformation Under Load of from 0.3 to 0.6 millimeters.

12

18. The absorbent composite according to any preceding claim except claim 10 or 11, wherein the superabsorbent material has a Wicking Index of about 10 centimeters or greater.

19. An absorbent composite according to any of claims 1 to 17, wherein the superabsorbent material has a Wicking Index of about 12 centimeters or greater.

20. An absorbent composite according to any preceding claim, wherein the superabsorbent material has a Wicking Index of about 15 centimeters or greater.

21. An absorbent composite according to any preceding claim, wherein the superabsorbent material has a Wicking Index of about 18 centimeters or greater.

22. An absorbent composite according to any preceding claim except claim 10 comprising about 40 weight percent or more superabsorbent material.

23. An absorbent composite according to any preceding claim comprising about 50 weight percent or more superabsorbent material.

24. An absorbent composite according to any preceding claim except claim 11 comprising about 60 weight percent or more superabsorbent material.

25. An absorbent composite according to any preceding claim except claim 11 comprising about 70 weight percent or more superabsorbent material.

26. An absorbent composite according to any preceding claim except claim 11 comprising about 80 weight percent or more superabsorbent material.

27. An absorbent composite according to any preceding claim except claim 11 comprising about 90 weight percent or more superabsorbent material.

28. An absorbent composite according to any preceding claim, wherein the superabsorbent material has an Absorbent Capacity of about 28 grams per gram or greater.

29. An absorbent composite according to any preceding claim, wherein the superabsorbent material has an Absorbent Capacity of about 32 grams per gram or greater.

30. An absorbent composite according to any preceding claim, wherein the superabsorbent material has an Absorbent Capacity of about 36 grams per gram or greater.

31. An absorbent composite according to any preceding claim, wherein the superabsorbent material has an Absorbent Capacity of about 40 grams per gram or greater.

32. An absorbent article comprising a liquid-permeable facing material, a liquid impermeable backing material, and an absorbent composite according to any preceding claim sandwiched between the facing material and the backing material.

33. An absorbent article according to claim 32 in the form of a disposable diaper.

34. An absorbent article according to claim 32 in the form of a training pant.

35. An absorbent article according to claim 32 in the form of an incontinence garment.

36. An absorbent article according to claim 32 in the form of a bed pad.

**Patentansprüche**

1. Saugfähiger Verbundwerkstoff, umfassend eine Matrix aus Fasern und superabsorbierendem Material, der wenigstens etwa 30 Gewichtsprozent superabsorbierendes Material, beruhend auf dem kombinierten Gewicht der Fasern und des superabsorbierenden Materials aufweist, dadurch gekennzeichnet, dass das superabsorbierende Material einen Dochtwirkungsparameter von etwa 700 oder mehr aufweist.

2. Saugfähiger Verbundwerkstoff gemäß Anspruch 1, wobei das superabsorbierende Material einen Dochtwirkungsparameter von etwa 800 oder mehr aufweist.

3. Saugfähiger Verbundwerkstoff gemäß Anspruch 1 oder 2, wobei das superabsorbierende Material einen Dochtwirkungsparameter von etwa 850 oder mehr aufweist.

4. Saugfähiger Verbundwerkstoff gemäß einem der vorangegangenen Ansprüche, wobei das superabsorbierende Material einen Dochtwirkungsparameter von etwa 900 oder mehr aufweist.

5. Saugfähiger Verbundwerkstoff gemäß einem der vorangegangenen Ansprüche, wobei das superabsorbierende Material eine Saugfähigkeit unter Beanspruchung (AUL) von etwa 13 oder mehr aufweist.

6. Saugfähiger Verbundwerkstoff gemäß einem der vorangegangenen Ansprüche, wobei das superabsorbierende Material eine Saugfähigkeit unter Beanspruchung von etwa 17 oder mehr aufweist.

7. Saugfähiger Verbundwerkstoff gemäß Anspruch 6, wobei das superabsorbierende Material einen Dochtwirkungsindex von etwa 10 Zentimeter oder mehr aufweist.

8. Saugfähiger Verbundwerkstoff gemäß Anspruch 6 oder 7, wobei das superabsorbierende Material eine Verformung unter Beanspruchung (DUL) von etwa 0,6 Millimeter oder weniger aufweist.

9. Saugfähiger Verbundwerkstoff gemäß einem der vorangegangenen Ansprüche, wobei das superabsorbierende Material eine Verformung unter Beanspruchung von etwa 0,6 Millimeter oder weniger, und einen Dochtwirkungsindex von etwa 10 Zentimeter aufweist.

10. Saugfähiger Verbundwerkstoff gemäß einem der vorangegangenen Ansprüche, umfassend wenigstens 50 Gewichtsprozent superabsorbierendes Material, beruhend auf dem kombinierten Gewicht der Fasern und des superabsorbierenden Materials, wobei das superabsorbierende Material eine Verformung unter Beanspruchung von etwa 0,6 Millimeter oder weniger, und einen Dochtwirkungsindex von etwa 12 Zentimeter oder mehr aufweist.

11. Saugfähiger Verbundwerkstoff gemäß einem der Ansprüche 1 bis 9, umfassend 30 bis 60 Gewichtsprozent superabsorbierendes Material, beruhend auf dem kombinierten Gewicht der Fasern und des superabsorbierenden Materials, wobei das superabsorbierende Material eine Saugkapazität von 28 bis 41 Gramm pro Gramm, eine Verformung unter Beanspruchung von 0,3 bis 0,6 Millimeter und einen Dochtwirkungsindex von 12 bis 19 Zentimeter aufweist.

12. Saugfähiger Verbundwerkstoff gemäß einem der vorangegangenen Ansprüche, wobei das superabsorbierende Material eine Saugfähigkeit unter Beanspruchung von etwa 20 oder mehr aufweist.

13. Saugfähiger Verbundwerkstoff gemäß einem der vorangegangenen Ansprüche, wobei das superabsorbierende Material eine Saugfähigkeit unter Beanspruchung von etwa 25 oder mehr aufweist.

14. Saugfähiger Verbundwerkstoff gemäß einem der vorangegangenen Ansprüche, wobei das superabsorbierende Material eine Verformung unter Beanspruchung von etwa 0,5 Millimeter oder weniger aufweist.

15. Saugfähiger Verbundwerkstoff gemäß einem der vorangegangenen Ansprüche, wobei das superabsorbierende Material eine Verformung unter Beanspruchung von etwa 0,4 Millimeter oder weniger aufweist.

16. Saugfähiger Verbungdwerkstoff gemäß einem der vorangegangenen Ansprüche außer Anspruch 11, wobei das superabsorbierende Material eine Verformung unter Beanspruchung von etwa 0,3 Millimeter oder weniger aufweist.

17. Saugfähiger Verbundwerkstoff gemäß einem der Ansprüche 1 bis 15, wobei das superabsorbierende Material eine Verformung unter Beanspruchung von 0,3 bis bis 0,6 Millimeter aufweist.

18. Saugfähiger Verbundwerkstoff gemäß einem der vorangegangenen Ansprüche außer Anspruch 10 oder 11, wobei das superabsorbierende Material einen Dochtwirkungsindex von etwa 10 Zentimeter oder mehr aufweist.

19. Saugfähiger Artikel gemäß einem der Ansprüche 1 bis 17, wobei das superabsorbierende Material einen Dochtwirkungsindex von etwa 12 Zentimeter oder mehr aufweist.

20. Saugfähiger Artikel gemäß einem der vorangegangenen Ansprüche, wobei das superabsorbierende Material einen Dochtwirkungsindex von etwa 15 Zentimeter oder mehr aufweist.

21. Saugfähiger Verbundwerkstoff gemäß einem der vorangegangenen Ansprüche, wobei das superabsorbierende Material einen Dochtwirkungsindex von etwa 18 Zentimeter oder mehr aufweist.

22. Saugfähiger Verbundwerkstoff gemäß einem der vorangegangenen Ansprüche außer Anspruch 10, umfassend etwa 40 Gewichtsprozent oder mehr superabsorbierendes Material.

23. Saugfähiger Verbundwerkstoff gemäß einem der vorangegangenen Ansprüche, umfassend etwa 50 Gewichtsprozent oder mehr superabsorbierendes Material.

24. Saugfähiger Verbundwerkstoff gemäß einem der vorangegangenen Ansprüche außer Anspruch 11, umfassend etwa 60 Gewichtsprozent oder mehr superabsorbierendes Material.

25. Saugfähiger Verbundwerkstoff gemäß einem der vorangegangenen Ansprüche außer Anspruch 11, umfassend etwa 70 Gewichtsprozent oder mehr superabsorbierendes Material.

26. Saugfähiger Verbundwerkstoff gemäß einem der vorangegangenen Ansprüche außer Anspruch 11, umfassend etwa 80 Gewichtsprozent oder mehr superabsorbierendes Material.

27. Saugfähiger Verbundwerkstoff gemäß einem der vorangegangenen Ansprüche außer Anspruch 11, umfassend etwa 90 Gewichtsprozent oder mehr superabsorbierendes Material.

28. Saugfähiger Verbundwerkstoff gemäß einem der vorangegangenen Ansprüche, wobei das superabsorbierende Material eine Saugkapazität von etwa 28 Gramm pro Gramm oder mehr aufweist.

29. Saugfähiger Verbundwerkstoff gemäß einem der vorangegangenen Ansprüche, wobei das superabsorbierende Material eine Saugkapazität von etwa 32 Gramm pro Gramm oder mehr aufweist.

30. Saugfähiger Verbundwerkstoff gemäß einem der vorangegangenen Ansprüche, wobei das superabsorbierende Material eine Saugkapazität von etwa 36 Gramm pro Gramm oder mehr aufweist.

31. Saugfähiger Verbundwerkstoff gemäß einem der vorangegangenen Ansprüche, wobei das superabsorbierende Material eine Saugkapazität von etwa 40 Gramm pro Gramm oder mehr aufweist.

32. Saugfähiger Artikel, umfassend ein wasserdurchlässiges Deckmaterial, ein wasserundurchlässiges Unterlagenmaterial und einen saugfähigen Verbundwerkstoff gemäß einem der vorangegangenen Ansprüche, der zwischen dem Deckmaterial und dem Unterlagenmaterial sandwichartig angeordnet ist.

33. Saugfähiger Artikel gemäß Anspruch 32 in Form einer Wegwerfwindel.

34. Saugfähiger Artikel gemäß Anspruch 32 in Form einer Trainingshose.

35. Saugfähiger Artikel gemäß Anspruch 32 in Form eines Inkontinenzkleidungsstücks.

36. Saugfähiger Artikel gemäß Anspruch 32 in Form einer Betteinlage.

**Revendications**

1. Composite absorbant comprenant une matrice de fibres et un matériau superabsorbant, renfermant au moins environ 30% en poids de matériau superabsorbant sur la base du poids combiné des fibres et de matériau superabsorbant, caractérisé en ce que le matériau superabsorbant a un Paramètre de Drainage d'environ 700 ou plus.

2. Composite absorbant selon la revendication 1, dans lequel le matériau superabsorbant a un Paramètre de Drainage d'environ 800 ou plus.

3. Composite absorbant selon la revendication 1 ou 2, dans lequel le matériau superabsorbant a un Paramètre de Drainage d'environ 850 ou plus.

4. Composite absorbant selon l'une quelconque des revendications précédentes, dans lequel le matériau superabsorbant a un Paramètre de Drainage d'environ 900 ou plus.

5. Composite absorbant selon l'une quelconque des revendications précédentes, dans lequel le matériau superabsorbant a une Capacité d'Absorption sous Charge d'environ 13 ou plus.

6. Composite absorbant selon l'une quelconque des revendications précédentes, dans lequel le matériau superabsorbant a une Capacité d'Absorption sous Charge d'environ 17 ou plus.

7. Composite absorbant selon la revendication 6, dans lequel le matériau superabsorbant a un Indice de Drainage d'environ 10 centimètres ou plus.

8. Composite absorbant selon la revendication 6 ou 7, dans lequel le matériau superabsorbant a une Déformation sous Charge d'environ 0,6 millimètre ou moins.

9. Composite absorbant selon l'une quelconque des revendications précédentes, dans lequel le matériau superabsorbant a une Déformation sous Charge d'environ 0,6 millimètre ou moins et un Indice de Drainage d'environ 10 centimètres ou plus.

10. Composite absorbant selon l'une quelconque des revendications précédentes, comprenant au moins environ 50% en poids de matériau superabsorbant sur la base du poids combiné des fibres et du matériau superabsorbant, le matériau superabsorbant ayant une Déformation sous Charge d'environ 0,6 millimètre ou moins et un Indice de Drainage d'environ 12 centimètres ou plus.

11. Composite absorbant selon l'une quelconque des revendications 1 à 9, comprenant entre 30 et 60% en poids de matériau superabsorbant sur la base du poids combiné des fibres et du matériau superabsorbant, le matériau superabsorbant ayant une Capacité d'Absorption sous Charge comprise entre 28 et 41 grammes/gramme, une Déformation sous Charge comprise entre 0,3 et 0,6 mm et un Indice de Drainage compris entre 12 et 19 centimètres.

12. Composite absorbant selon l'une quelconque des revendications précédentes, dans lequel le matériau superabsorbant a une Capacité d'Absorption sous Charge d'environ 20 ou plus.

13. Composite absorbant selon l'une quelconque des revendications précédentes, dans lequel le matériau superabsorbant a une Capacité d'Absorption sous Charge d'environ 25 ou plus.

14. Composite absorbant selon l'une quelconque des revendications précédentes, dans lequel le matériau superabsorbant a une Déformation sous Charge d'environ 0,5 millimètre ou moins.

15. Composite absorbant selon l'une quelconque des revendications précédentes, dans lequel le matériau superabsorbant a une Déformation sous Charge d'environ 0,4 millimètre ou moins.

16. Composite absorbant selon l'une quelconque des revendications précédentes, à l'exception de la revendication 11, dans lequel le matériau superabsorbant a une Déformation sous Charge d'environ 0,3 millimètre ou moins.

17. Composite absorbant selon l'une quelconque des revendications 1 à 15, dans lequel le matériau superabsorbant a une Déformation sous Charge comprise entre 0,3 et 0,6 millimètre.

18. Composite absorbant selon l'une quelconque des revendications précédentes, à l'exception de la revendication 10 ou 11, dans lequel le matériau superabsorbant a un Indice de Drainage d'environ 12 centimètres ou plus.

19. Composite absorbant selon l'une quelconque des revendications 1 à 17, dans lequel le matériau superabsorbant a un Indice de Drainage d'environ 12 centimètres ou plus.

20. Composite absorbant selon l'une quelconque des revendications précédentes, dans lequel le matériau superabsorbant a un Indice de Drainage d'environ 15 centimètres ou plus.

21. Composite absorbant selon l'une quelconque des revendications précédentes, dans lequel le matériau superabsorbant a un Indice de Drainage d'environ 18 centimètres ou plus.

22. Composite absorbant selon l'une quelconque des revendications précédentes, à l'exception de la revendication 10, comprenant environ 40% en poids ou plus de matériau superabsorbant.

23. Composite absorbant selon l'une quelconque des revendications précédentes, comprenant environ 50% en poids ou plus de matériau superabsorbant.

24. Composite absorbant selon l'une quelconque des revendications précédentes, à l'exception de la revendication 11, comprenant environ 60% en poids ou plus de matériau superabsorbant.

25. Composite absorbant selon l'une quelconque des revendications précédentes, à l'exception de la revendication 11, comprenant environ 70% en poids ou plus de matériau superabsorbant.

26. Composite absorbant selon l'une quelconque des revendications précédentes, à l'exception de la revendication 11, comprenant environ 80% en poids ou plus de matériau superabsorbant.

27. Composite absorbant selon l'une quelconque des revendications précédentes, à l'exception de la revendication 11, comprenant environ 90% en poids ou plus de matériau superabsorbant.

28. Composite absorbant selon l'une quelconque des revendications précédentes, dans lequel le matériau superabsorbant a une Capacité d'Absorption d'environ 28 grammes par gramme ou plus.

29. Composite absorbant selon l'une quelconque des revendications précédentes, dans lequel le matériau superabsorbant a une Capacité d'Absorption d'environ 32 grammes par gramme ou plus.

30. Composite absorbant selon l'une quelconque des revendications précédentes, dans lequel le matériau superabsorbant a une Capacité d'Absorption d'environ 36 grammes par gramme ou plus.

31. Composite absorbant selon l'une quelconque des revendications précédentes, dans lequel le matériau superabsorbant a une Capacité d'Absorption d'environ 40 grammes par gramme ou plus.

32. Article absorbant comprenant un matériau perméable aux liquides tourné vers la source de liquide, un matériau support imperméable aux liquides, et un composite absorbant selon l'une quelconque des revendications précédentes, pris en sandwich entre le matériau tourné vers la source de liquide et le matériau support.

33. Article absorbant selon la revendication 32, qui se présente sous la forme d'un change jetable pour nourrisson.

34. Article absorbant selon la revendication 32, qui se présente sous la forme d'une culotte d'apprentissage de la propreté.

35. Article absorbant selon la revendication 32, qui se présente sous la forme d'un vêtement pour adulte incontinent.

36. Article absorbant selon la revendication 32, qui se présente sous la forme d'une alèse.

FIG. I

FIG. 4

FIG. 3

FIG. 2

FIG. 5

FIG. 6

EP 0 761 191 B1

FIG. 7

FIG. 8